# EUROPÄISCHE PATENTSCHRIFT

(11) **EP 1 750 656 B1**
(45) Veröffentlichungstag und Bekanntmachung des Hinweises auf die Patenterteilung: **01.07.2015**
(21) Anmeldenummer: 05750506.7
(22) Anmeldetag: 17.05.2005
(51) Int. Cl.: A61K 8/34, A61K 8/64, A61K 8/81, A61Q 5/04, A61Q 5/06

(54) **Verfahren zur temporären Haarfestigung und -formung mittels eines temperaturbeständigen Stylingmittels**
Process for styling hair with temperature-resistant styling medium
Procédé de remodelage de la coiffure utilisant des produits de mise en forme thermorésistants

(30) Priorität: 18.05.2004 WO PCT/EP2004/005317
(43) Veröffentlichungstag der Anmeldung: 14.02.2007
(73) Patentinhaber: Henkel AG & Co. KGaA, 40589 Düsseldorf (DE)
(72) Erfinder: HENTRICH, Dirk, 22457 Hamburg (DE); EMMERLING, Winfried, 25436 Tornesch (DE); FLODROP, Helga, Van, 21075 Hamburg (DE)
(86) Internationale Anmeldenummer: PCT/EP2005/005338
(87) Internationale Veröffentlichungsnummer: WO 2005/112878

(56) Entgegenhaltungen:
- EP-A- 0 715 842
- WO-A-89/04653
- WO-A1-02/060397
- WO-A1-2004/024176
- US-A- 2 390 073
- US-A- 4 374 125
- US-A- 6 056 946
- US-A1- 2002 182 163
- US-B1- 6 241 977

## Beschreibung

Die vorliegende Erfindung betrifft die Verwendung von kosmetischen Zubereitungen enthaltend ausschließlich Inhaltsstoffe, die bis zu 200 °C gegenüber thermischer Zersetzung stabil sind zur Formgebung der Haare und ein Verfahren zur temporären Haarverformung.

Keratinische Fasern, insbesondere menschliche Haare, werden heutzutage einer Vielzahl von Behandlungen unterzogen. Dabei spielen Behandlungen, die zu einer permanenten oder temporären Formgebung der Haare dienen, um ansprechend aussehende Frisuren zu erhalten, eine wichtige Rolle. Aufgrund von aktuellen Modeströmungen gelten immer wieder Frisuren als chic, die sich bei vielen Haartypen nur unter Verwendung bestimmter festigender Wirkstoffe aufbauen bzw. für einen längeren Zeitraum aufrecht erhalten lassen. Als temporäre Formgebungen kommen beispielsweise Gestaltungen wie Lockung, Glättung, Toupierung oder auch Festigung in Frage. Temporäre Formgebungen, die einen guten Halt ergeben sollen, ohne das gesunde Aussehen der Haare, wie z.B. deren Glanz, zu beeinträchtigen, können beispielsweise durch Stylingmittel, wie Haarsprays, Haarwachse, Haargele, Haarfestiger, Fönwellen, Stylingsprays etc. erzielt werden. Das temporäre Formgeben wird auch als Hair-Styling oder Styling bezeichnet, Formgebungsmittel werden auch als Stylingmittel bezeichnet.

Gleichfalls spielt im Bereich der temporären Formgebung der Haare die Haarverformung unter Wärmeeinwirkung z.B. durch einen Fön, einen Lockenstab oder ein Glätteisen eine wichtige Rolle. Zur Verbesserung von Stylingergebnissen wird oftmals vor einer Wärmeanwendung ein Stylingmittel, z.B. ein Haarfestiger oder ein Stylingspray, im Falle höherer Temperaturen auch ein sogenanntes thermisches Stylingspray auf das Haar aufgetragen. Besonders im Friseurbereich finden sogenannte Glätteisen ("hot irons") immer mehr Verwendung. Glätteisen weisen zwei parallel angeordnete Metall- oder Keramikplatten auf, durch welche die Haare, nach Aufheizen der Platten, hindurchgezogen werden, indem das Glätteisen entlang einer Haarsträhne geführt wird. Handelsübliche Glätteisen lassen sich auf Temperaturen im Bereich von 150-250 °C aufheizen. Ziel der Glätteisenanwendung ist es, gewelltes bis gelocktes Haar thermisch/physikalisch zu glätten. Sollen Haare durch ein Glätteisen geglättet werden, wird auf das Haar üblicherweise vorher als Stylingmittel für Glätteisen, ein thermisches Stylingspray, auch Glätteisenspray bezeichnet, aufgetragen. Das Spray unterstützt dabei das Gleiten des Eisens sowie die Glättung des Haares.

Auch um den gegenteiligen Effekt, nämlich die Wellung oder Kräuselung von glattem Haar zu erreichen, wird die Haarverformung unter Wäremeeinwirkung eingesetzt. Anstelle eines Glätteisens kommt in diesem Fall ein Lockenstab oder auch ein Föhn mit Diffusor zum Einsatz. Dabei wird das glatte Haar um den aufgeheizten Lockenstab oder den Diffusor gewickelt, wobei wiederum Temperaturen bis hin zu 250°C erreicht werden. Auch in diesem Fall kommen zur Verbesserung des Stylingergebnisses in der Regel thermische Stylingsprays zum Einsatz, die vor der eigentlichen Temperaturbehandlung auf das Haar aufgebracht werden.

Diese die Formgebung unterstützenden Zusammensetzungen enthalten unter anderem neben haarpflegenden Wirkstoffen und haarfestigenden Wirkstoffen, bei denen es sich in der Regel um polymere Wirkstoffe handelt, zur angenehmeren Anwendung auch Parfüme. Die in diesen Zusammensetzungen enthaltenen Substanzen sind den extremen Wärmebedingungen, insbesondere bei Haarbehandlungen mit Glätteisen oder Lockenstäben die oftmals Temperaturen von bis zu 250 °C erreichen, ausgesetzt.

Aus dem Stand der Technik sind zahlreiche kosmetische Zusammensetzungen zur temporären Haarverformung auch zur Anwendung unter Wärmeeinwirkung bekannt.

US2002/0182163 und US6056946 offenbaren Verfahren zur temporären Haarfestigung und -formung durch Anwendung von Glätteisen.

Nachteil der aus dem Stand der Technik bekannten kosmetischen Zusammensetzungen zur temporären Haarverformung unter Wärmeeinwirkung ist deren intensive unangenehme Geruchsbildung bei Wärmeanwendung, die beim Anwender oftmals einen leichten Hustenreiz während der Wärmebehandlung des Haares auslösen kann. Es besteht der Verdacht, dass aus dem Stand der Technik bekannte sogenannte thermische Stylingsprays, das heißt formgebende Sprays, die zur Anwendung auf dem Haar unter Wärmeeinwirkung von bis zu 150-250 °C bestimmt sind, bei diesen Wärmebedingungen die eine oder andere toxikologisch nicht unbedenkliche Substanz freisetzen, die der Anwender einatmet. Dies kann gesundheitliche Beeinträchtigungen mit sich bringen, die insbesondere für im Friseursalon tätige Personen relevant sind, da diese häufig mehrmals täglich thermische Haarverformungen unter Verwendung von thermischen Stylingsprays bzw. Stylingmitteln für Glätteisen vornehmen.

Es war somit Aufgabe der vorliegenden Erfindung, ein sicheres Verfahren zur Behandlung der Haare unter Wärmeeinwirkung bereitzustellen, das heißt ein Verfahren mit einer toxikologisch unbedenklichen Zusammensetzung, die auch bei Anwendungstemperaturen von bis zu 200°C toxikologisch unbedenklich bleibt. Es war weiter Aufgabe der Erfindung, ein Verfahren zur Behandlung der Haare unter Wärmeeinwirkung bei Temperaturen im Bereich von bis zu 200 °C bereitzustellen, die in der Anwendung bei diesen Temperaturen eine gegenüber herkömmlichen Zusammensetzungen deutlich reduzierte Geruchsbildung aufweist und damit in der Anwendung wesentlich angenehmer ist.

Diese Aufgaben werden durch ein Verfahren zur temporären Haarfestigung und -formung mit einer kosmetischen Zusammensetzung gemäß Anspruch 1, die ausschließlich Inhaltsstoffe enthält, die bis zu mindestens 200 °C gegenüber thermischer Zersetzung stabil sind, in hervorragender Weise gelöst.

Die Erfindung betrifft daher ein Verfahren zur temporären Haarfestigung und -formung mit einer kosmetischen Zusammensetzung gemäß Anspruch 1, die ausschließlich Inhaltsstoffe enthält, die bis zu mindestens 200 °C gegenüber thermischer Zersetzung stabil sind, wobei die Zusammensetzung auf das feuchte oder trockene Haar aufgebracht wird und das Haar unter Wärmeeinwirkung von bis zu 200°C geformt wird, wobei die Formung des Haares in einer Glättung besteht und die Hitzeeinwirkung durch ein Glätteisen erfolgt. Die abhängigen Ansprüche stellen bevorzugte Ausführungsformen dieses Erfindungsgegenstandes dar.

Ein weiterer Gegenstand der Erfindung ist die Verwendung dieser Zusammensetzungen zur temporären Formgebung der Haare, im Rahmen einer Glättung durch Hitze ein wirkung mit einem Glätteisen.

Im Rahmen der der vorliegenden Erfindung zugrundliegenden Untersuchungen wurde überraschend gefunden, dass kosmetische Zusammensetzungen, die ausschließlich Inhaltsstoffe enthalten, die bis zu mindestens 200 °C gegenüber thermischer Zersetzung stabil sind, bei der Verwendung zur temporären Formgebung der Haare als Stylingmittel für Glätteisen, keine unangenehme Geruchsbildung verursachen und sehr angenehm und sicher in der Anwendung sind.

In Untersuchungen wurde festgestellt, dass die Inhaltsstoffe von aus dem Stand der Technik bekannten thermischen Stylingsprays bei Wärmebedingungen von bis zu 150-250 °C nicht stabil sind, sondern sich unter Bildung auch gasförmiger Zersetzungsprodukte zersetzen wie z.B. das Glycerin. Beispielsweise ist über das Glycerin, welches sich z.B. beim Erhitzen von Pflanzenölen in der Pfanne auf etwa 200 °C durch Spalten der Öle in Glycerin und Fettsäuren bildet, bekannt, dass es sich beim weiteren Erhitzen zersetzt und zu einer stechenden Geruchsbildung führt. Des weiteren besteht die Vermutung, dass sich auch Parfüme bei Temperaturen von unter 200 °C zersetzen und zu einer unangenehmen Geruchsbildung führen.

Die gasförmigen Zersetzungsprodukte gelangen in die Atemluft der Anwender und können einen Hustenreiz auslösen, sowie zu einer Beeinträchtigung des Wohlbefindens führen.

Dieses Problem der Zersetzung der Inhaltsstoffe von thermischen Stylingsprays unter den üblichen Anwendungstemperaturen einer Behandlung mit einem Glätteisen ist im Stand der Technik nicht beschrieben. Die Inhaltsstoffe von aus dem Stand der Technik bekannten thermischen Stylingsprays unterscheiden sich nicht von den Inhaltsstoffen der Stylingsprays, die zur Haarverformung bei niedrigen Temperaturen von bis zu 80 °C vorgesehen sind. Bei der Entwicklung der aus dem Stand der Technik bekannten thermischen Stylingsprays wurde offenbar den extremen Wärmebedingungen bei einer Glätteisenanwendung nicht Rechnung getragen, sondern übliche Inhaltsstoffe bekannter Stylingsprays auch für Glätteisensprays eingesetzt, unabhängig von ihrer Stabilität gegenüber den höheren Temperaturen.

Die Zusammensetzungen in den Verfahren gemäß der vorgeleglen Ansprüche führen zu einer deutlichen Erhöhung der Sicherheit bei der Anwendung von Stylingmitteln z.B. bei der Glätteisenbehandlung sowie zu einer angenehmeren Handhabung.

Überraschenderweise wurde festgestellt, dass diese Zusammensetzungen darüber hinaus bei Glätteisenanwendungen eine gegenüber den aus dem Stand der Technik bekannten thermischen Stylingsprays deutlich verbesserte Haltbarkeit der Glättung bewirken.

In den dieser Erfindung zugrundeliegenden Untersuchungen wurde die Zersetzungstemperatur der Inhaltsstoffe mittels dynamischer Differentialkalorimetrie (Differential Scanning Calorimetry, DSC) als erste exotherme Abweichung einer DSC-Messung unter Luftatmosphäre von einer DSC-Messung unter Stickstoff-Atmosphäre bestimmt. Die Messungen wurden unter Standardbedingungen auf einem Universal V3.8 B TA Instruments Gerät bzw. einem Universal V3.9 A TA Instruments Gerät mit einer Heizgeschwindigkeit von 20 °C/min, einer Probeneinwaage von 6-7 mg und einer Stickstoff- bzw. Luftzufuhr von 3 l/h in einem offenen Aluminiumtiegel durchgeführt. Die in den erfindungsgemäßen Zusammensetzungen enthaltenen Inhaltsstoffe, wie z.B. Proteinhydrolysate, Polymere, Tenside, Silikonöle oder Aromen weisen alle eine nach obiger Methode bestimmte Zersetzungstemperatur auf, die oberhalb von 200 °C liegt. Die Erfindung ist aber nicht auf Zusammensetzungen beschränkt, die Inhaltsstoffe enthalten, die eine nach obiger Methode bestimmte Zersetzungstemperatur von über 200 °C aufweisen. Sie umfasst auch Zusammensetzungen, deren Inhaltsstoffe eine nach anderen Methoden, wie z.B. der Kapillarmethode, bestimmte Zersetzungstemperatur von über 200 °C aufweisen.

Lediglich beispielhaft sind im folgenden die DSC-Messungen an bevorzugten Inhaltsstoffen aufgezeigt, wie z.B. an dem Proteinhydrolysat Promois Silk (Figur 1), an den filmbildenden Polymeren Luviquat FC 370 (Figur 2), Luviquat Style (Figur 3), Luviquat Hold (Figur 4), Styleze W-20 (Figur 5), PVP/VA-Copolymer (Figur 6), dem Silikonöl Baysilone Öl PH 300 (Figur 7) und dem Aromastoff Phenethylalkohol (Figur 8).

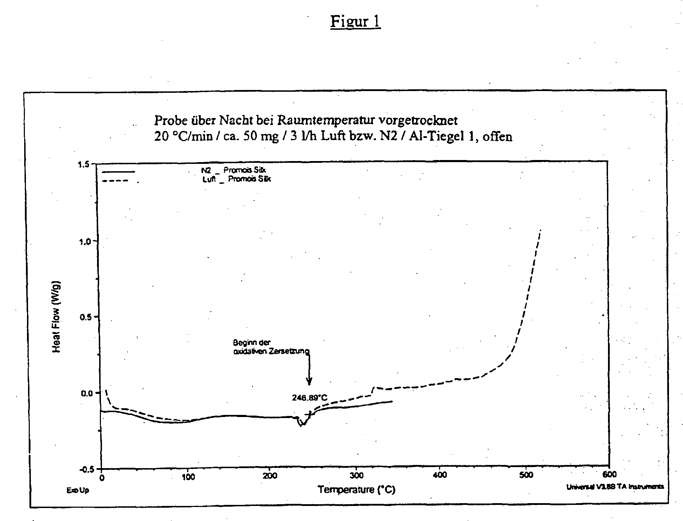

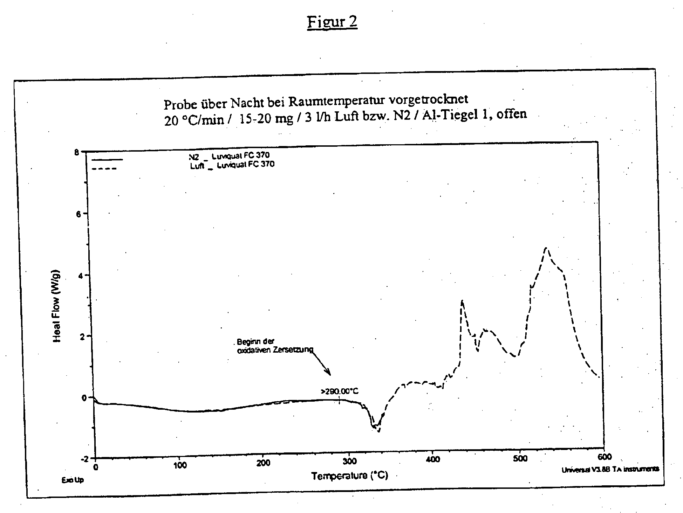

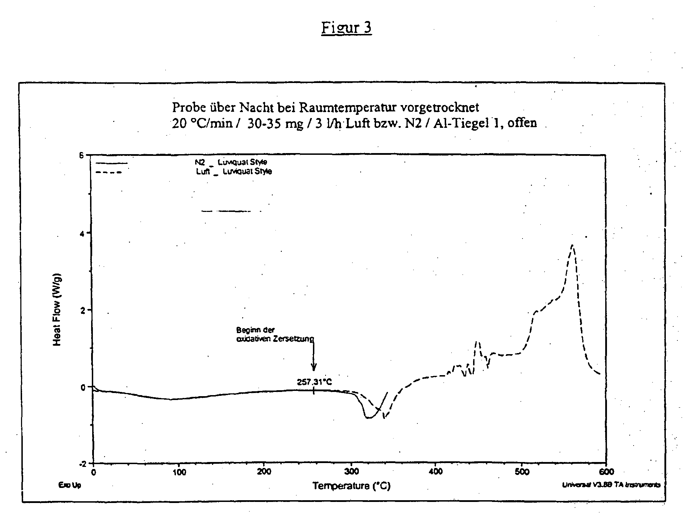

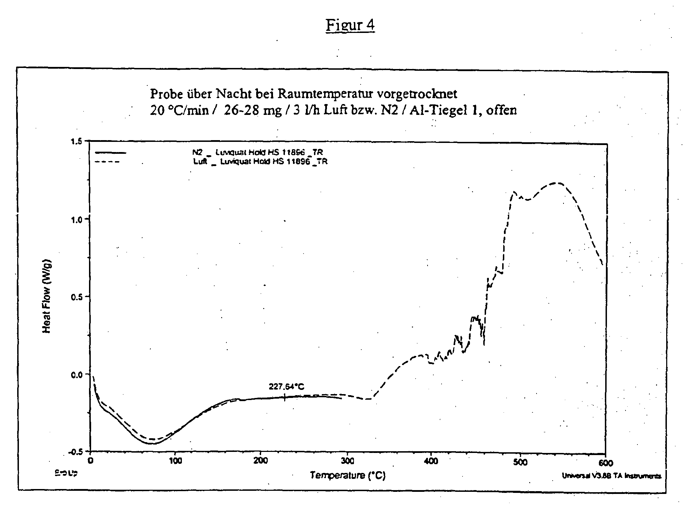

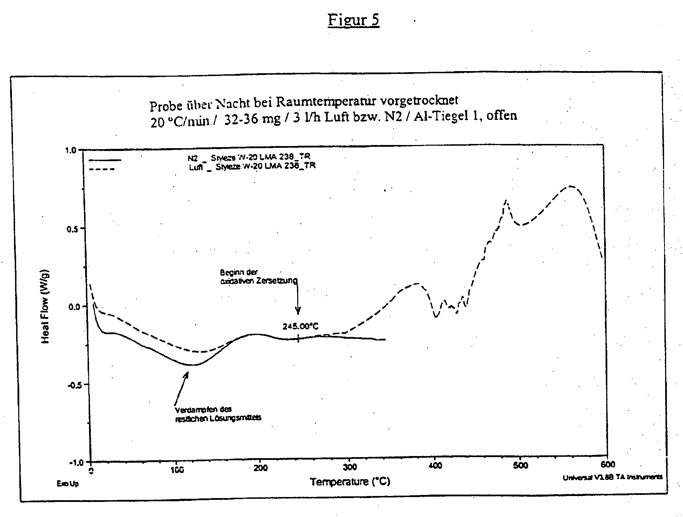

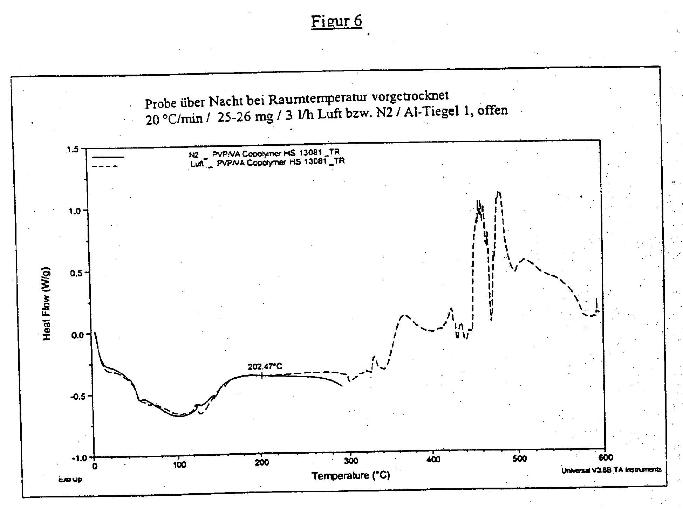

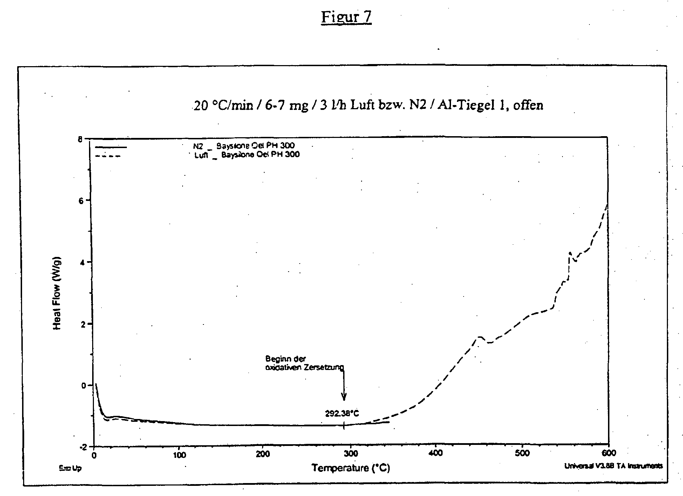

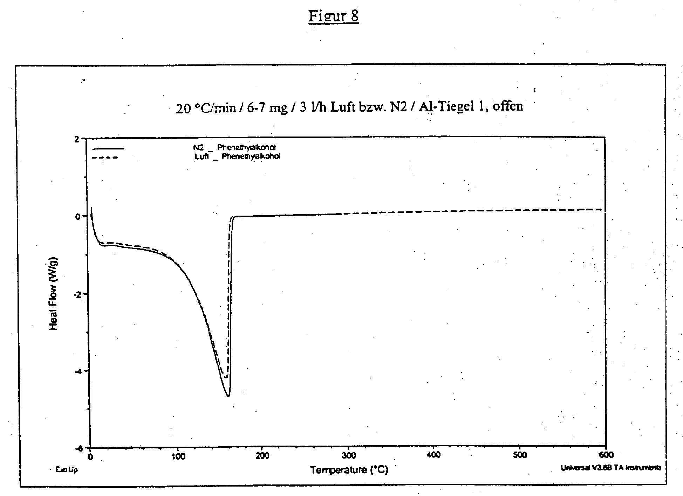

Während in den Figuren 1-7 die durch exotherme Abweichung der beiden zum einen unter Luftatmosphäre und zum anderen unter Stickstoffatmosphäre gemessenen DSC-Kurven bestimmte Zersetzungstemperatur über 200 °C liegt, tritt gemäß den DSC-Kurven der Figur 8 keine Zersetzung des Aromastoffs Phenethylalkohol ein. Phenethylalkohol verdampft zersetzungsfrei bei etwa 170 °C

Die verwendeten Zusammensetzungen enthalten vorzugsweise sowohl haarpflegende als auch dem Haar Form gebende Wirkstoffe. Erfindungsgemäß müssen diese Wirkstoffe bis zu 200°C gegenüber thermischer Zersetzung stabil sein.

Die verwendeten Haarbehandlungsmittel enthalten als Lösungsmittel Wasser und/oder Ethanol.

Als haarpflegende Komponenten werden in den Zusammensetzungen Proteinhydrolysate eingesetzt, sofern diese bis zu 200°C gegenüber thermischer Zersetzung stabil sind. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Seiden-Proteinhydrolysate, die auch in Form von Salzen vorliegen können. Sie werden beispielsweise unter der Marke "Promois^{®} (Interorgana)" vertrieben. Ein erfindungsgemäß bevorzugtes temperaturstabiles tierische Proteinhydrolysat ist Promois Silk 1000.

Pflanzliche Proteinhydrolysate sind beispielsweise Weizenproteinhydrolysate, welche erfindungsgemäß bevorzugte pflanzliche Proteinhydrolysate sind.

Die Proteinhydrolysate oder deren Derivate sind in den erfindungsgemäß verwendeten Zubereitungen bevorzugt in Mengen von 0,1-10 Gew.-% bezogen auf das gesamte Mittel enthalten. Mengen von 0,1-5 Gew.-% sind besonders bevorzugt.

Die verwendeten Zusammensetzungen enthalben temperaturstabile kationische Polymere.

Unter kationischen Polymeren sind Polymere zu verstehen, welche in der Haupt- und/oder Seitenkette Gruppen aufweisen, welche "temporär" oder "permanent" kationisch sein können. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (III), 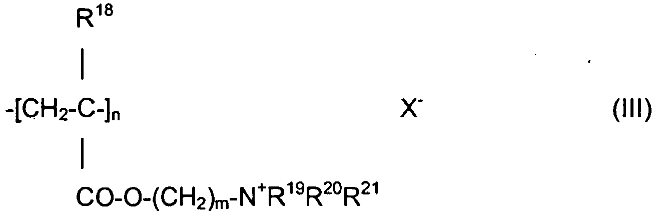 in der R¹⁸ = -H oder -CH₃ ist, R¹⁹, R²⁰ und R²¹ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (III) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R¹⁸ steht für eine Methylgruppe
- R¹⁹, R²⁰ und R²¹ stehen für Methylgruppen
- m hat den Wert 2.

Als physiologisch verträgliche Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann, falls gewünscht, mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwässrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (III) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethyl-ammoniumchlorid-Copolymer. Solche Copolymere, bei denen die Monomere in einem Gewichtsverhältnis von etwa 20:80 vorliegen, sind im Handel als ca. 50 %ige nichtwässrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationisierter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905, HM 552 und Hold angeboten werden.
- quaternierter Polyvinylalkohol,
sowie die unter den INCI-Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind. Ihre Herstellung ist z.B. in DE 44 40 625 A1 und in DE 1 95 03 465 A1 beschrieben.

Besonders gut geeignete Chitosane weisen einen Deacetylierungsgrad von wenigstens 80 % und ein Molekulargewicht von 5 · 10⁵ bis 5 · 106 (g/mol) auf.

Zur Herstellung erfindungsgemäß zu verwendender Zubereitungen muss das Chitosan in die Salzform überführt werden. Dies kann durch Auflösen in verdünnten wässrigen Säuren erfolgen. Als Säuren sind sowohl Mineralsäuren wie z.B. Salzsäure, Schwefelsäure und Phosphorsäure als auch organische Säuren, z.B. niedermolekulare Carbonsäuren, Polycarbonsäuren und Hydroxycarbonsäuren geeignet. Weiterhin können auch höhermolekulare Alkylsulfonsäuren oder Alkylschwefelsäuren oder Organophosphorsäuren verwendet werden, soweit diese die erforderliche physiologische Verträglichkeit aufweisen. Geeignete Säuren zur Überführung des Chitosans in die Salzform sind z.B. Essigsäure, Glycolsäure, Weinsäure, Apfelsäure, Zitronensäure, Milchsäure, 2-Pyrrolidinon-5-carbonsäure, Benzoesäure oder Salicylsäure. Bevorzugt werden niedermolekulare Hydroxycarbonsäuren wie z.B. Glycolsäure oder Milchsäure verwendet.

Bevorzugte kationische, filmbildende Polymere sind die Vinylpyrrolidon-Methoimidazoliniumchlorid-Copolymere, wie sie unter der Bezeichnung "Luviquat^{®}" angeboten werden, z.B. Luviquat FC 370 (INCI-Bezeichnung: Polyquaternium-16), Luviquat Style (INCI-Bezeichnung: Polyquaternium-16) und Luviquat Hold (INCI-Bezeichnung: Polyquaternium-46), sowie das unter der INCI-Bezeichnung Polyquarternium-55 bekannte kationische Terpolymer aus Vinylpyrrolidon, Dimethylaminopropylmethacrylamid und Lauryldimethylpropylmethacrylamidoammoniumchlorid, welches unter der Bezeichnung Styleze W erhältlich ist.

Kationische Polymere sind in den erfindungsgemäß verwendeten Zusammensetzungen bevorzugt in Mengen von 0,2-4 Gew.-%, bevorzugt 0,5-2 Gew.-% enthalten.

Des weiteren können in den erfindungsgemäß verwendeten Zusammensetzungen temperaturstabile nichtionogene Polymere enthalten sein.

Geeignete nichtionogene Polymere sind beispielsweise:
- Vinylpyrrolidon/Vinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) vertrieben werden.
- Schellack.
- Polyvinylpyrrolidone, wie sie beispielsweise unter der Bezeichnung Luviskol^{®} (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder HydroxyGruppen enthalten.
- Glycosidisch substituierte Silicone gemäß der EP 0 612 759 B1.

Erfindungsgemäß bevorzugt ist z.B. das wasserlösliche, filmbildende Vinylpryrrolidon/Vinylacetat-Copolymer, erhältlich unter der Handelsbezeichnung PVP/VA W-635 (INCI-Bezeichnung: VP/VA Copolymer).

Die nichtionogenen Polymere sind in den erfindungsgemäßen Zubereitungen bevorzugt in Mengen von 0,5-10 Gew.-%, besonders bevorzugt in Mengen von 1-5 Gew.-% enthalten.

Weiterhin können die erfindungsgemäß Verwendeten Zusammensetzungen temperaturstabile Tenside enthalten.

Unter dem Begriff Tenside werden grenzflächenaktive Substanzen, die an Ober- und Grenzflächen Adsorptionsschichten bilden oder in Volumenphasen zu Mizellkolloiden oder lyotropen Mesophasen aggregieren können, verstanden. Man unterscheidet Aniontenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wässriger Lösung stark hydratisiert sind. Weitergehende Definitionen und Eigenschaften von Tensiden finden sich in "H.-D.Dörfler, Grenzflächen- und Kolloidchemie, VCH Verlagsgesellschaft mbH. Weinheim, 1994". Die zuvor wiedergegebene Begriffsbestimmung findet sich ab S. 190 in dieser Druckschrift.

Insbesondere geeignet sind temperaturstabile kationische Tenside vom Typ der quaternären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryltrimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierte Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-palmitoyloxyethyl)dimethylammoniumchlorid, sowie Dehyquart^{®} F-75, Dehyquart^{®} C-4046, Dehyquarf^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Erfindungsgemäß bevorzugt eingesetzt wird das Cetyltrimethylammoniumchlorid, das unter der Bezeichnung Genamin CTAC erhältlich ist.

Die kationischen Tenside sind in den erfindungsgemäßen Zusammensetzungen bevorzugt in Mengen von 0,1-1 Gew.-%, besonders bevorzugt 0,2-0,6 Gew.-% enthalten.

Weiterhin können als konditionierende Wirkstoffe geeignete Silikonöle, wie z.B. Baysilone Öl PH 300, in den erfindungsgemäß verwendeten Zusammensetzungen enthalten sein.

In den erfindungsgemäß verwendeten Zusammensetzungen sind Silikonöle bevorzugt in Mengen von 0,1-1 Gew.-%, besonders bevorzugt in Mengen von 0,1-0,5 Gew.-% enthalten.

Als Duftstoff werden in den erfindungsgemäßen Zusammensetzungen nicht Parfümöle mit einer oftmals nicht exakt bekannten Zusammensetzung und einer Zersetzungstemperatur von unter 200 °C eingesetzt, sondern es können bestimmte toxikologisch unbedenkliche Aromastoffe eingesetzt werden, die zersetzungsfrei verdunsten. Erfindungsgemäß bevorzugte Aromastoffe sind 2-Phenylethanol, der einen Rosenduft vermittelt und bei ca. 160 °C verdunstet, und (2-Phenylethyl)-acetat, das einen Siedepunkt bei 101,3 kPa von 232,6 °C aufweist.

Bezüglich weiterer üblicher Inhaltsstoffe wird ausdrücklich auf die dem Fachmann bekannten Monographien, beispielsweise K. Schrader, Grundlagen und Rezepturen der Kosmetiker, Dr. Alfred Hütig-Verlag, Heidelberg, verwiesen.

Die erfindungsgemäß verwendeten Haarbehandlungsmittel können in den üblichen Applikationsformen, wie z.B. als Sprühlösung, als Aerosolspray, als Schaum oder Schüttlotion formuliert werden. Erfindungsgemäß bevorzugt ist die Formulierung als Sprühlösung.

Die folgenden Ausführungsbeispiele verdeutlichen die Erfindung, ohne sie zu beschränken.

### Beispiel 1 - Sprühlösung

| | |
|---|---|
| Entsalztes Wasser | 73,38 Gew.-% |
| Weizenproteinhydrolysat kationisiert | 0,6 Gew.-% |
| Promois Silk 1000¹) | 0,5 Gew.-% |
| Ethanol vergällt 96 Vol.-% | 20,0 Gew.-% |
| Ortho-Phosphorsäure | 0,02 Gew.-% |
| Genamin CTAC²) | 0,5 Gew.-% |
| PVP/VA W-635³⁾ | 5,0 Gew.-% |

| | |
|---|---|
| ¹⁾ SeidenproteinHydrolysat; INCI-Bezeichnung: Hydrolyzed Silk ²⁾ Trimethylhexadecylammoniumchlorid (ca. 29 Gew.-% Aktivsubstanz in Wasser); INCI-Bezeichnung: Cetrimonium Chloride ³⁾ Vinylpyrrolidon-Vinylacetat-Copolymer, stabilisiert mit 0,05 Gew.-% Dodecyltrimethylammoniumchlorid; INCI-Bezeichnung: VP/VA Copolymer | |

### Beispiel 2 - Sprühlösung

| | |
|---|---|
| Entsalztes Wasser | 68,47 Gew.-% |
| Promois Silk 1000 | 1,0 Gew.-% |
| Pearlpurin PP⁴⁾ | 0,01 Gew.-% |
| Ethanol vergällt 96 Vol.-% | 20,0 Gew.-% |
| Ortho-Phosphorsäure | 0,02 Gew.-% |
| Genamin CTAC | 0,5 Gew.-% |
| PVP/VA W-635 | 10,0 Gew.-% |

| | |
|---|---|
| ⁴⁾ Mischung aus einem Stärkehydrolysat und gemahlenen Süßwasserperlen; INCI-Bezeichnung: Maltodextrin and Pearl Powder | |

## Patentansprüche

1. Verfahren zur temporären Haarfestigung bzw. -formung, wobei eine kosmetische Zusammensetzung auf das feuchte oder trockene Haar aufgebracht wird und das Haar unter Wärmeeinwirkung von bis zu 200 °C geformt wird, wobei die Formung des Haares in einer Glättung besteht und die Hitzeeinwirkung durch ein Glätteisen erfolgt, wobei die kosmetische Zusammensetzung enthält
• ausschließlich Inhaltsstoffe, die bis zu mindestens 200 °C gegenüber thermischer Zersetzung stabil sind,
• als Lösungsmittel Wasser und/oder Ethanol,
• ein oder mehrere Proteinhydrolysate,
• einen oder mehrere konditionierende Wirkstoffe aus der Gruppe der kationischen Tenside, kationischen Polymere, Alkylamidoaminen und synthetischen Öle,
• ein oder mehrere filmbildende Polymere aus der Gruppe der Vinylpyrrolidon/Methylimidazoliniumchlorid-Copolymere, der VinylpyrrolidonNinylacetat-Copolymere und der kationischen Terpolymere aus Vinylpyrrolidon, Dimethylaminopropylmethacrylamid und Lauryldimethylpropylmethacrylamidoammoniumchlorid, und
• ein oder mehrere Silikonöle,
**dadurch gekennzeichnet, dass** die Inhaltsstoffe eine durch exotherme Abweichung einer DSC-Messung des Inhaltsstoffes unter Luftatmosphäre zu einer DSC-Messung unter Stickstoffatmosphäre bestimmte Zersetzungstemperatur von höher als 200 °C haben.

2. Verfahren nach Anspruch 1, **dadurch gekennzeichnet, dass** die kosmetische Zusammensetzung ein oder mehrere Aromastoffe enthält.

3. Verwendung einer kosmetischen Zusammensetzung zur temporären Formgebung der Haare, wobei die Formgebung der Haare unter Wärmeeinwirkung von bis zu 200 °C im Rahmen einer Glättung durch ein Glätteisen erfolgt, wobei die kosmetische Zusammensetzung enthält
• ausschließlich Inhaltsstoffe, die bis zu mindestens 200 °C gegenüber thermischer Zersetzung stabil sind,
• als Lösungsmittel Wasser und/oder Ethanol,
• ein oder mehrere Proteinhydrolysate,
• einen oder mehrere konditionierende Wirkstoffe aus der Gruppe der kationischen Tenside, kationischen Polymere, Alkylamidoaminen und synthetischen Öle,
• ein oder mehrere filmbildende Polymere aus der Gruppe der Vinylpyrrolidon/Methylimidazoliniumchlorid-Copolymere, der Vinylpyrrolidon/Vinylacetat-Copolymere und der kationischen Terpolymere aus Vinylpyrrolidon, Dimethylaminopropylmethacrylamid und Lauryldimethylpropylmethacrylamidoammoniumchlorid, und
• ein oder mehrere Silikonöle,
**dadurch gekennzeichnet, dass** die Inhaltsstoffe eine durch exotherme Abweichung einer DSC-Messung des Inhaltsstoffes unter Luftatmosphäre zu einer DSC-Messung unter Stickstoffatmosphäre bestimmte Zersetzungstemperatur von höher als 200 °C haben.

## Claims

1. Method for temporary hair setting and/or styling, wherein a cosmetic composition is applied to the wet or dry hair and the hair is styled while applying heat of up to 200°C, wherein the styling of the hair consists of straightening and the heat is applied by a straightening iron, wherein the cosmetic composition contains
• only ingredients that are stable up to at least 200°C with respect to thermal decomposition,
• water and/or ethanol as the solvent,
• one or more protein hydrolyzates,
• one or more conditioning active ingredients from the group of cationic surfactants, cationic polymers, alkylamidoamines and synthetic oils,
• one or more film-forming polymers from the group of vinylpyrrolidone/methylimidazolinium chloride copolymers, vinylpyrrolidone/vinyl acetate copolymers and cationic terpolymers of vinylpyrrolidone, dimethylaminopropyl methacrylamide and lauryl dimethylpropyl methacrylamidoammonium chloride, and
• one or more silicone oils,
**characterized in that** the ingredients have a certain decomposition temperature higher than 200°C as determined by an exothermic deviation of a DSC measurement of the ingredient under an air atmosphere to a DSC measurement under a nitrogen atmosphere.

2. Method according to claim 1, **characterized in that** the cosmetic composition contains one or more aromatic substances.

3. Use of a cosmetic composition for temporary styling of hair, wherein the hair is styled under the influence of heat of up to 200°C, within the context of straightening using a straightening iron, wherein the cosmetic composition contains
• only ingredients that are stable up to at least 200°C with respect to thermal decomposition,
• water and/or ethanol as the solvent,
• one or more protein hydrolyzates,
• one or more conditioning active ingredients from the group of cationic surfactants, cationic polymers, alkylamidoamines and synthetic oils,
• one or more film-forming polymers from the group of vinylpyrrolidone/methylimidazolinium chloride copolymers, vinylpyrrolidone/vinyl acetate copolymers and cationic terpolymers of vinylpyrrolidone, dimethylaminopropyl methacrylamide and lauryl dimethylpropyl methacrylamidoammonium chloride, and
• one or more silicone oils,
**characterized in that** the ingredients have a certain decomposition temperature higher than 200°C as determined by an exothermic deviation of a DSC measurement of the ingredient under an air atmosphere to a DSC measurement under a nitrogen atmosphere.

## Revendications

1. Procédé de mise en forme ou de fixation temporaire des cheveux, dans lequel une composition cosmétique est appliquée sur les cheveux humides ou secs et les cheveux sont mis en forme sous l'effet de la chaleur jusqu'à 200°C, la mise en forme des cheveux consistant en un lissage et l'effet de la chaleur étant apporté par un fer à lisser, la composition cosmétique contenant
• exclusivement des ingrédients qui sont stables jusqu'à au moins 200°C vis-à-vis de la décomposition thermique,
• de l'eau et/ou de l'éthanol comme solvant,
• un ou plusieurs hydrolysats de protéines,
• un ou plusieurs agents de conditionnement choisis dans le groupe des agents tensioactifs cationiques, des polymères cationiques, des alkylamidoamines et des huiles de synthèse,
• un ou plusieurs polymères filmogènes choisis dans le groupe des copolymères vinylpyrrolidone/chlorure de méthylimidazolinium, des copolymères vinylpyrrolidone/acétate de vinyle et des terpolymères cationiques choisis parmi le vinylpyrrolidone, le diméthylaminopropylméthacrylamide et le chlorure de lauryldiméthylpropylméthacrylamidoammonium et
• une ou plusieurs huiles de silicone,
**caractérisé en ce que** les ingrédients ont une température de décomposition supérieure à 200°C, déterminée par déviation exothermique d'une mesure DSC (calorimétrie différentielle à balayage) de l'ingrédient sous une atmosphère d'air par rapport à une mesure DSC sous atmosphère d'azote.

2. Procédé selon la revendication 1, **caractérisé en ce que** la composition cosmétique contient un ou plusieurs aromes.

3. Utilisation d'une composition cosmétique pour la mise en forme temporaire des cheveux, dans laquelle la mise en forme des cheveux est effectuée sous l'effet de la chaleur jusqu'à 200°C par un lissage avec un fer à lisser, la composition cosmétique contenant
• exclusivement des ingrédients qui sont stables jusqu'à au moins 200°C vis-à-vis de la décomposition thermique,
• de l'eau et/ou de l'éthanol comme solvant,
• un ou plusieurs hydrolysats de protéines,
• un ou plusieurs agents de conditionnement choisis dans le groupe des agents tensioactifs cationiques, des polymères cationiques, des alkylamidoamines et des huiles de synthèse,
• un ou plusieurs polymères filmogènes choisis dans le groupe des copolymères vinylpyrrolidone/chlorure de méthylimidazolinium, des copolymères vinylpyrrolidone/acétate de vinyle et des terpolymères cationiques choisis parmi le vinylpyrrolidone, le diméthylaminopropylméthacrylamide et le chlorure de lauryldiméthylpropylméthacrylamidoammonium et
• une ou plusieurs huiles de silicone,
**caractérisé en ce que** les ingrédients ont une température de décomposition supérieure à 200°C, déterminée par déviation exothermique d'une mesure DSC (calorimétrie différentielle à balayage) de l'ingrédient sous une atmosphère d'air par rapport à une mesure DSC sous atmosphère d'azote.
